# FASCICULE DE BREVET EUROPEEN

(11) **EP 3 273 941 B1**
(45) Date de publication et mention de la délivrance du brevet: **17.06.2020**
(21) Numéro de dépôt: 16714801.4
(22) Date de dépôt: 23.03.2016
(51) Int. Cl.: A61K 9/00, A61K 9/70, A61N 1/04, A61N 1/30, A61N 1/32

(54) **MATRICE POLYMÉRIQUE ADHÉSIVE POUR IONTOPHORÈSE ET DISPOSITIF POUR L'IONTOPHORÈSE COMPRENANT LADITE MATRICE**
KLEBENDE POLYMERMATRIX FÜR IONTOPHORESE UND VORRICHTUNG FÜR IONTOPHORESE MIT DIESER MATRIX
ADHESIVE POLYMER MATRIX FOR IONTOPHORESIS AND DEVICE FOR IONTOPHORESIS INCLUDING SAID MATRIX

(30) Priorité: 24.03.2015 FR 1552414
(43) Date de publication de la demande: 31.01.2018
(73) Titulaire: Feeligreen, 06130 Grasse (FR)
(72) Inventeur: BIANCHI, Christophe, 06100 Nice (FR); GRAVELINES, Elodie, 06160 Juan les Pins (FR)
(74) Mandataire: Bourrières, Patrice
(86) Numéro de dépôt international: PCT/EP2016/056336
(87) Numéro de publication internationale: WO 2016/150994

(56) Documents cités:
- GB-A- 410 009
- US-A- 4 731 926
- US-A- 5 169 383

## Description

### DOMAINE DE L'INVENTION

L'invention concerne une matrice polymérique adhésive pour iontophorèse et un dispositif pour l'iontophorèse comprenant ladite matrice.

L'invention concerne les techniques consistant à utiliser un courant électrique pour faciliter la diffusion transdermique de substances actives, et concerne plus particulièrement un dispositif d'iontophorèse.

### ETAT DE LA TECHNIQUE

L'iontophorèse est une technique qui peut être utilisée à des fins cosmétiques et/ou médicales pour introduire des substances actives dans le derme de la peau au moyen d'un courant.

Les dispositifs d'iontophorése comprennent des réservoirs ou des patchs contenant les substances actives à administrer. Ces réservoirs ou patchs sont appliqués entre le dispositif et la peau de l'utilisateur. L'objectif de ces dispositifs est d'entrainer par le courant électrique les substances actives du réservoir ou du patch vers le derme. Le courant électrique doit circuler entre les électrodes au travers du derme. Or, avec les dispositifs actuels, très peu de courant circule réellement au travers du derme. De ce fait l'administration de la substance active est réduite.

Il existe donc le besoin de proposer une solution qui permette d"améliorer la délivrance des substances actives au derme.

Le document US 5,169,383 décrit un dispositif comprenant les tampons d'électrode séparés par un isolant. Le dispositif comprend une couche adhésive supplémentaire.

### RESUME DE L'INVENTION

A cet effet la présente invention concerne une matrice polymérique adhésive pour iontophorése avec une première face configurée pour être appliquée sur la peau et une deuxième face opposée configurée pour coopérer avec des électrodes. La matrice comprend des zones conductrices électriquement et au moins une zone isolante électriquement, chaque zone conductrice est isolée électriquement d'une autre zone conductrice par une zone isolante.

Une telle matrice permet d'assurer un passage optimal du courant au travers du derme. En effet, le courant circulant entre deux électrodes de polarités opposées ne peut que passer par le derme, il n'y a pas de passage conducteur entre deux zones conductrices au travers de la matrice. De cette manière la matrice selon l'invention permet de supprimer tout courant de fuite parasite de l'efficacité de l'iontophorèse. Le courant circule entre deux zones conductrices au travers de la peau entrainant ainsi au moins un principe actif à administrer.

Selon un autre aspect, l'invention concerne un dispositif d'iontophorèse comprenant des électrodes et une source d'énergie ainsi qu'une matrice telle que décrite ci-dessus.

### BREVES DESCRIPTION DES FIGURES

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.

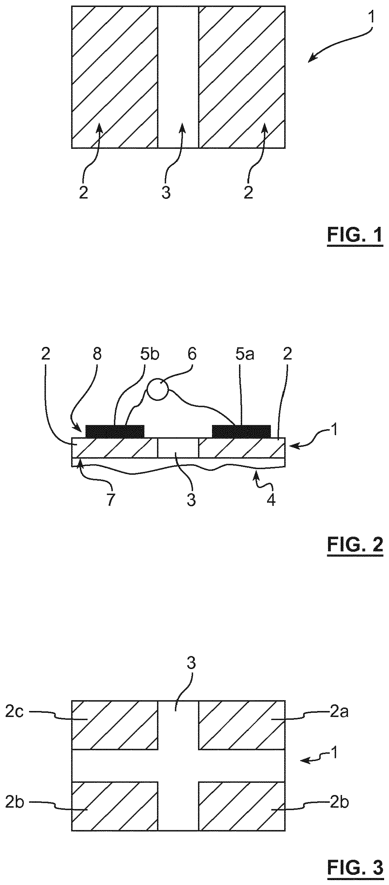

La figure 1 représente en vue de dessus un exemple de disposition des zones conductrices et isolante d'une matrice selon l'invention.
La figure 2 représente en vue en coupe le dispositif selon l'invention appliqué sur la peau.
La figure 3 représente en vue de dessus un autre exemple de disposition des zones conductrices et isolante d'une matrice selon l'invention.

### DESCRIPTION DETAILLEE DE L'INVENTION

Avant d'entamer une revue détaillée de modes de réalisation de l'invention, sont énoncées ci-après des caractéristiques optionnelles qui peuvent éventuellement être utilisées en association ou alternativement.

On rappelle tout d'abord que l'invention porte sur une matrice polymérique adhésive par la délivrance de principe actif pour iontophorèse présentant une première face destinée à être appliquée sur la peau et une deuxième face destinée à coopérer avec des électrodes caractérisée en ce qu'elle comprend des zones conductrices électriquement et au moins une zone isolante électriquement, chaque zone conductrice étant isolée d'une autre zone conductrice par une zone isolante.

Avantageusement, la matrice selon l'invention est telle que les zones conductrices comprennent au moins un principe actif à délivrer.

Avantageusement, les zones conductrices sont configurées pour coopérer avec des électrodes fournissant un courant électrique, le au moins un principe actif de chaque zone conductrice étant choisi en fonction de la polarisation de l'électrode coopérant avec ladite zone.

Avantageusement, les zones conductrices comprennent des principes actifs différents.

Avantageusement, la au moins une zone isolante présente une largeur minimale de 1mm séparant deux zones conductrices.

Avantageusement, les zones s'étendent sur toute l'épaisseur de la matrice.

Avantageusement, la matrice comprend au moins un polymère, au moins un adhésif et au moins un plastifiant ;

Avantageusement, la matrice comprend un promoteur d'absorption.

L'invention concerne également un dispositif pour l'iontophorèse comprenant au moins deux électrodes et une source d'énergie connectée électriquement aux électrodes, caractérisé en qu'il comprend une matrice polymérique adhésive. Avantageusement, les zones conductrices de la matrice polymérique adhésive sont reliées aux électrodes.

L'invention concerne une matrice polymérique adhésive pour l'iontophorèse. La matrice 1 comprend une première face 7 et une deuxième face 8 opposée. La première face 7 est destinée à être appliquée sur la peau de l'utilisateur, avantageusement au niveau de la zone à traiter. La deuxième face 8 est configurée pour coopérer avec un dispositif d'iontophorèse et plus particulièrement avec des électrodes 5a, 5b.

Le dispositif d'iontophorèse comprend des électrodes 5a, 5b et une source d'énergie 6. Le dispositif peut se présenter sous différente forme que ce soit uniquement des électrodes reliées électriquement à une source d'énergie déportée ou sous forme d'un boitier ou de patch tels que décrits dans des demandes de brevet précédentes du demandeur. Avantageusement, le dispositif comprend un module électronique, avantageusement des moyens d'activation tels que par exemple un bouton de commande. Le module électronique comprend avantageusement des moyens de commande et des moyens de mesures de paramètres physiologiques par exemple de mesure de la température, du pH, de la pression, de l'humidité relative, de la résistivité.

Le module électronique est avantageusement destiné à contrôler le courant fourni aux électrodes 5a, 5b. Le module électronique permet avantageusement de contrôler l'intensité, la tension et/ou la durée d'administration. Selon une possibilité, le module électronique comprend un microprocesseur.

Selon une possibilité, la source d'énergie 6 est une source d'énergie électrique telle qu'un accumulateur rechargeable ou une pile permettant l'application d'une tension aux électrodes 5a, 5b par exemple par l'intermédiaire d'un générateur de tension. La source d'énergie 6 peut être diverse. Avantageusement, la source d'énergie 6 est autonome, à titre d'exemple un générateur autonome d'énergie par récupération d'énergie peut être utilisé ou bien des batteries de type piles.

Selon un mode de réalisation avantageux, le module électronique comprend la source de d'énergie, préférentiellement une source de courant avantageusement autonome. Cette disposition est particulièrement avantageuse pour permettre un usage ambulatoire du dispositif selon l'invention.

Le dispositif est avantageusement configuré pour délivrer un courant d'intensité contrôlée. A titre préféré, l'intensité I est inférieure ou égale à 1mA.

Les électrodes 5a, 5b permettent de transmettre un courant électrique à la peau 4 en traversant la matrice 1 avantageusement suivant son épaisseur et plus particulièrement des zones conductrices 2 décrites ci-après.

Le dispositif de l'invention comprend avantageusement un élévateur de tension. L'élévateur de tension est avantageusement présent dans le module électronique. L'élévateur de tension permet d'élever la tension fournie aux électrodes à partir de la tension délivrée par la source d'énergie.

Selon un mode de réalisation préféré, la matrice 1 est séparable du dispositif, c'est-à-dire amovible. Préférentiellement, le dispositif est réutilisable. La matrice 1 est-elle avantageusement un consommable qui va être utilisée une seule fois ou quelque fois mais dans une mesure plus faible que le dispositif. La matrice est jetable. Ceci est particulièrement intéressant d'un point de vue écologique et économique car le dispositif comprend les électrodes 5a, 5b.

Le dispositif selon l'invention permet d'avoir un dispositif qui puisse à la fois être à usage unique ou du moins utilisé pour un seul patient tout en réutilisant les éléments électroniques coûteux.

Selon l'invention, la matrice 1 est une nappe souple. La souplesse est telle que deux points opposés du pourtour peuvent être accolés, sans détérioration de la matrice 1. Cette souplesse est avantageusement maintenue lorsque le dispositif est connecté et fixé sur la matrice 1.

Préférentiellement, la matrice 1 présente une épaisseur de 25 à 60 µm. La souplesse de la matrice permet de se conformer à la zone d'application du corps où elle est destinée à être appliquée.

Le dispositif et la matrice 1 selon l'invention sont utilisables sur l'ensemble des zones du corps humain facilement et sans gêne ou désagrément pour l'utilisateur.

La matrice 1 comprend au moins principe actif destiné à être administré à la peau 4 de l'utilisateur. On entend par principe actif une substance ayant un effet thérapeutique ou cosmétique sur l'utilisateur.

La matrice 1 selon l'invention comprend des zones conductrices 2 électriquement et au moins une zone isolante électriquement3. De cette manière, les zones conductrices 2 sont isolées les unes de autres par au moins une zone isolante 3. Avantageusement, les zones conductrices 2 coopèrent avec des électrodes 5a, 5b d'un dispositif d'iontophorèse.

Les zones conductrices 2 sont configurées pour permettre une conduction électrique entre les électrodes 5a 5b du dispositif et la peau sur laquelle est appliquée la matrice 1. Le courant électrique traverse la matrice 1 de la deuxième face 8 vers la première face 7 c'est-à-dire suivant l'épaisseur de la matrice 1.

Les zones isolantes 3 sont configurées pour permettre une isolation à la conduction électrique, notamment entre les zones conductrices 2.

A titre d'exemple, la présence de principe actif ionisé dans une zone conductrice 2 de la matrice 1 suffit à apporter une propriété de conduction électrique à ladite zone. A contrario, l'absence de principe actif ionisé et préférentiellement d'autre élément ionisé dans la zone isolante 3 suffit à apporter une propriété de résistance à la conduction électrique à ladite zone.

Suivant le procédé de fabrication de la matrice 1 décrit ci-après, la matrice 1 comprend un film par exemple de silicone constituant la base de la matrice 1. Ce film est noyé dans le polymère de la matrice polymérique.

Les zones conductrices 2 et isolante 3 sont formées sur toute l'épaisseur de la matrice 1

Préférentiellement, les zones conductrices 2 contiennent au moins un principe actif. Préférentiellement, les zones isolantes 1 ne comprennent pas de principe actif.

Selon une possibilité préférée, les principes actifs de chaque zone conductrice 2 sont choisis en fonction de la polarisation de l'électrode 5a, 5b coopérant avec ladite zone.

Avantageusement, les électrodes sont choisies pour avoir au moins une électrode de polarité opposée à au moins une autre, préférentiellement de polarité opposée deux à deux, de sorte à permettre une circulation du courant électrique d'une zone conductrice 2 à une autre zone conductrice 2 au travers de la peau.

Une zone isolante 3 séparant au moins deux zones conductrices 2 présentent une largeur minimale de 1mm de sorte à assurer une isolation satisfaisante

Selon un mode de réalisation, la matrice 1 comprend une pluralité de zones conductrices 2a, 2b, 2c, 2d... séparées par au moins une zone isolante 3. Chaque zone conductrice comprend un principe actif différent choisi en fonction de la polarisation de l'électrode 5 coopérant. Selon une possibilité, la matrice 1 est configurée pour permettre une iontophorèse séquentielle. C'est-à-dire que les principes actifs de chaque zone conductrice 2 sont administrés séquentiellement. Le dispositif est configuré pour fournir du courant aux zones conductrices 2 par les électrodes 5, de manière séquentielle ce qui permet d'administrer des principes actifs les uns après les autres de sorte à améliorer les traitements.

Chaque zone conductrice 2a, 2b, 2c et 2d est respectivement reliée à au moins une électrode 5a, 5b.

A titre d'exemple, dans une zone conductrice 2 configurée pour coopérer avec une électrode 5a de type cathode, les principes actifs sont choisis parmi la liste ci-après au regard desquels sont indiqués des exemples de pathologies, symptômes ou indications pour lesquels ces principes actifs pourraient être utilisés :
- pénicilline
- sulfamides 10% : furoncles
- acide nicotinique
- iodure de potassium 1 à 3% : anti-scléreux, tonique vasculaire, anti-arthrosique, tonique nerveux, fibrolytique, sympatico-tonique, tonique vasculaire, cicatrice hypertrophique, sclérose artérielle, arthrose, arthrite, séquelle d'hémiplégie sans contracture, lésion nerveuse périphérique
- iodure de sodium 1 à 3% : sclérolytique, cicatrices adhérentes, chéloïdes, raideurs articulaires, maladie de Dupuytren
- salicylate de sodium 1 à 3% : anti-rhumatismal, antalgique, anti-œdèmateux, arthrose articulaire, PSH, névralgies, périphlébites
- soufre sublimé
- hyaluranidase 150 u 1 flacon : agent de diffusion, anti-œdémateux, œdèmes locaux, épanchements,lymphangite
- hydrocortisone 1% : anti-inflammatoire stéroïdien, inflammations rhumatismales sans signe d'ostéoporose
- succinate de prednisolone 1% : anti-inflammatoire stéroïdien, inflammations rhumatismales sans signe d'ostéoporose, anti-inflammatoire stéroïdien
- célestène : anti-inflammatoire stéroïdien, inflammations rhumatismales sans signe d'ostéoporose
- Beta-méthasone : anti-inflammatoire stéroïde + mucopolysaccharidase, arthrose, syndromes articulaires chroniques, tendinites, syndromes para-articulaires,maladies de Dupuytren et Lapeyronie, chéloïdes, tendinites
- percutalgine : anti-inflammatoire stéroïdien, inflammations rhumatismales sans signe d'ostéoporose, arthrite, syndrome articulaire
- ketoprofène :anti-inflammatoire non stéroïdien
- profénid 50 à associer à une mucopolysaccharidase : arthrose, syndrome para articulaire
- dicloflénac 7 : anti-inflammatoire non stéroïdien
- voltaren - à associer à une mucopolysaccharidase : syndromes ab-articulaires
- phénylbutazone : anti-inflammatoire non stéroïdien
- butazolidine : inflammations rhumatismales, inflammations post traumatiques
- salicylate de lithine 1% : anti-uricémique, syndromes goutteux et para-goutteux
- mucopolysaccharidase : agent de diffusion, cellulite, œdème, hématome, base pour pénétration des produits anti-inflammatoires
- idrocilamide : myorelaxant
- srilane : anti-inflammatoire
- brolitène : contractures musculaires, algies, tendinites
- thiomucase : anti-œdémateux, résolutif, lymphœdèmes, cellulite
- euclidan : anti-inflammatoire, vasodilatateur, algoneurodystrophie, circulation périphérique
- nicometat 2 amp : anti-inflammatoire, algoneurodystrophie
- alphamucase : anti-œdémateux, algoneurodystrophie
- chlorure de calcium : fibrolytique, cicatrices
- chlorure de sodium : fibrolytique, cicatrices
- acide thriodothryacétique : lipolytique, cellulite

A titre d'exemple, dans une zone conductrice 2 configurée pour coopérer avec une électrode 5b de type anode, les principes actifs sont choisis parmi la liste ci-après au regard desquels sont indiqués des exemples de pathologies, symptômes ou indications pour lesquels ces principes actifs pourraient être utilisés :
- carbaïne 5% : anesthésie locale, hyperalgies
- chlorproéthazine : décontracturant
- neuriplège : contractures, spasmophilie, myalgie
- tétanil : décontracturant, spasmophilie
- alphachymotrypsine : agent de diffusion, anti-œdémateux, contusions, entorses, œdèmes
- alphacutanée : agent de diffusion, anti-œdémateux, contusions, œdèmes
- thyroïde lyophilisée : lypolitique, catabolisant lipidique, obésité
- acide triodothryoacétique : lypolitique, obésité
- inflanil : anti-rhumatismal, arthrite, tendinite, syndrome articulaire
- silicium organique : régénérateur du tissu conjonctif, vergetures, cellulite localisée
- salicylate de lithine : anti-rhumatismal, arthrose des petites articulations
- adrénaline : vasoconstricteur, circulation périphérique
- nitrate d'aconitine : anti-névralgique, antalgique, névralgie du trijumeau, névralgie post-zostérienne
- percutalgine : antalgique, douleurs rachidiennes, algies ligamentaires, algies tendineuses
- apisine, apicure (venin d'abeille) : névralgie intercostale
- nitrate d'argent : anti-rhumatismal articulaire
- acétylcholine 0.5%
- vitamine B
- chlorure de calcium : sédative, recalcifiante, algies, hémiplégies spasmodique, ostéoporose, spasmophilie, algoneurodystrophies, raideurs articulaires
- chlorure de zinc : antiseptique, conjonctivite chronique, gynécologie, ORL
- sulfate de zinc : gynécologie, ORL
- chlorure de magnésium : sédatif, spasmolytique, spasmophilie, verrues planes
- sulfate de magnésium : spasmophilie, verrues planes
- chlorure d'ammonium : fibrolytique, raideurs articulaires
- flaxedyl : curarisant de synthèse, décontracturant, contractures, torticolis, dorsalgies, lombalgies, contractures musculaires rhumatismales, spasmophilie
- doryl 0.1%
- chlorydrate d'histamine : révulsif, hyperalgies, sciatalgie
- bichlorydrate d'histamine : révulsif, vasodilatateur, syndrome articulaire
- phosphate d'épinéphrine : vasoconstricteur, asthme, circulation périphérique
- sulfate de cuivre : antiseptique, fongicide, mycoses
- cocaïne
- procaïne
- novocaïne : anesthésie locale, antalgique, névralgie du trijumeau, zona
- citrate de potassium : spasmophilie, verrues planes
- percaïne 2 à 5%
- priscol 5 à 10%
- émanation de radium 100000 EM
- corticostéroïdes 1% : rhumatismes articulaires, (célestène, betnésol), goutte
- hydrocortisone 1% : rhumatismes articulaires, goutte
- histacone
- penicilline
- brome
- biomycine
- butazoline
- streptomyline

La matrice 1 comprend avantageusement au moins un polymère choisi parmi le polyuréthane, pour son élasticité, le polysiloxane pour sa capacité isolante, le Poly(méthyl méthacrylate) pour la force physique et la transparence, le Poly(vinyl alcool) pour son hydrophilie et sa force, le Polyéthylène pour sa dureté, sa solidité et son gonflement, le Polyvinylpyrrolidone pour ses capacités de suspension.

Préférentiellement, la matrice 1 est adhésive c'est-à-dire qu'elle est configurée pour coller ou adhérer, temporairement, à la peau sur laquelle elle est appliquée. A cet effet, la matrice 1 comprend au moins un adhésif choisi parmi l'acide acrylique, les polyacrylates, le polyisobutylène, le polyvinylpyrrolidone, les silicones.

Selon un mode de réalisation, la matrice 1 comprend au moins un plastifiant choisi parmi le sorbitol, le glycérol, le propylène glycol.

La matrice 1 comprend selon une possibilité un solvant.

La matrice 1 comprend suivant un mode de réalisation un promoteur d'absorption choisi eau, alcools, alcools gras tel que le propylène glycol, acides aminés, amides, esters, éthers tel que PEG, terpènes, terpènoïdes et huiles essentielles, sulfoxides, lipides.

Selon un mode de réalisation préféré, la matrice 1 est préparée suivant un procédé ci-après. Une solution d'adhésif est mélangée à une solution de polymère pour former un mélange A. Puis une solution comprenant un plastifiant et au moins un principe actif est ajouté au mélange A pour former le mélange B. Le mélange B est maintenu sous agitation lente, à une vitesse avantageusement inférieure à 200rpm, pendant une durée équivalente à une nuit. Le mélange est ensuite coulé sur au moins une portion d'un film par exemple de silicone.

La fabrication se fait par sérigraphie. Ces étapes sont répétées pour former une autre zone conductrice 2 et la zone isolante 3 respectivement sur des portions du même film par exemple de silicone. L'ensemble est séché en étuve par exemple pendant 30 minutes à 80°C. La matrice 1 obtenue est à conserver au dessiccateur.

Suivant le mode de réalisation dans lequel la matrice 1 comprend plusieurs zones conductrices 2a, 2b, 2c, 2d comprenant respectivement différents principes actifs. Les étapes décrites ci-dessus sont effectuées pour chacune des zones conductrices.

Le dispositif selon l'invention peut être utilisé dans des applications cosmétiques ou thérapeutiques. Si le dispositif est utilisé pour l'iontophorèse, il destiné à faire pénétrer des substances actives dans le derme, telles que par exemple la vitamine A ou rétinol ayant un effet dépigmentant, la lidocaïne ayant un effet anesthésique local, l'acide hyaluronique ayant des propriété régénératrices et cicatrisantes, l'acide rétinoïque pour le traitement de l'acné, la vitamine C ayant un effet antioxydant, un chélateur d'ions tel que l'acide β-alanine di-acétique pour le traitement des érythèmes, l'acide glycolique améliorant la texture de la peau, le phosphate sodique de dexaméthasone ayant un effet anti-inflammatoire ou tout autre type d'actif ionisé ou présenté sous forme d'émulsions anioniques ou cationiques afin de pouvoir être véhiculé par le courant.

Selon une possibilité avantageuse, le dispositif comprend une pluralité d'anodes et une pluralité de cathodes alternées avec les anodes. Dans cette configuration, le courant total se divise suivant plusieurs circuits anode-cathode de manière à ce que la densité du courant circulant d'une anode à une cathode soit préférentiellement inférieure à 1mA/cm2. En outre, afin d'empêcher la formation de lignes de champ externes, c'est-à-dire à l'extérieur du dispositif, les deux électrodes se trouvant aux deux extrémités sont de même polarité, c'est à dire que ce sont deux anodes ou deux cathodes.

### REFERENCES

- 1: Matrice
- 2: Zone conductrice
- 3: Zone isolante
- 4: Peau
- 5a. 5b.: Electrodes
- 6: Source d'énergie
- 7: Première face
- 8: Deuxième face

## Revendications

1. Matrice (1) polymérique adhésive pour la délivrance de principe actif par iontophorèse présentant une première face (7) destinée à être appliquée sur la peau et une deuxième face (8) destinée à coopérer avec des électrodes **caractérisée en ce qu'**elle comprend des zones conductrices (2) électriquement et au moins une zone isolante (3) électriquement, chaque zone conductrice (2) étant isolée d'une autre zone conductrice (2) par une zone isolante (3) et **en ce que** la matrice comprend un mélange comprenant au moins un polymère et au moins un adhésif.

2. Matrice (1) selon la revendication précédente dans laquelle ledit au moins un polymère est choisi parmi : le polyuréthane, le polysiloxane, le Poly(méthyl méthacrylate), le Poly(vinyl alcool), le Polyéthylène, le Polyvinylpyrrolidone.

3. Matrice (1) selon l'une quelconque des revendications précédentes dans laquelle ledit au moins un adhésif est choisi parmi : l'acide acrylique, les polyacrylates, le polyisobutylène, le polyvinylpyrrolidone, les silicones.

4. Matrice (1) selon l'une quelconque des revendications précédentes dans laquelle ledit mélange comprend au moins un plastifiant de préférence choisi parmi le sorbitol, le glycérol, le propylène glycol.

5. Matrice (1) selon l'une quelconque des revendications précédentes dans laquelle la matrice est une nappe souple.

6. Matrice (1) selon l'une quelconque des revendications précédentes dans laquelle les zones conductrices (2) comprennent au moins un principe actif à délivrer.

7. Matrice (1) selon la revendication précédente dans laquelle les zones conductrices (2) sont configurées pour coopérer avec des électrodes fournissant un courant électrique, le au moins un principe actif de chaque zone conductrice (2) étant choisi en fonction de la polarisation de l'électrode (5a, 5b) coopérant avec ladite zone.

8. Matrice (1) selon l'une quelconque des deux revendications précédentes dans lequel les zones conductrices (2) comprennent des principes actifs différents.

9. Matrice (1) selon l'une quelconque des revendications précédentes dans lequel la au moins une zone isolante (3) présente une largeur minimale de 1mm séparant deux zones conductrices (2).

10. Matrice (1) selon l'une quelconque des revendications précédente dans laquelle les zones s'étendent sur toute l'épaisseur de la matrice.

11. Matrice (1) selon l'une quelconque des revendications précédentes comprenant un promoteur d'absorption.

12. Dispositif pour l'iontophorèse comprenant au moins deux électrodes (5a, 5b) et une source d'énergie(6) connectée électriquement aux électrodes (5a, 5b) caractérisé en qu'il comprend une matrice (1) polymérique adhésive selon l'une quelconque des revendications précédentes.

13. Dispositif selon la revendication précédente dans lequel les zones conductrices (2) de la matrice (1) polymérique adhésive sont reliées aux électrodes (5a, 5b).

14. Procédé de fabrication d'une matrice selon l'une quelconque des revendications 1 à 11 comprenant au moins les étapes suivantes :
a. une solution d'adhésif est mélangée à une solution de polymère pour former un mélange A, puis
b. une solution comprenant un plastifiant et au moins un principe actif est ajoutée au mélange A pour former un mélange B,
c. le mélange B est ensuite coulé sur au moins une portion d'un film par exemple de silicone.

15. Procédé de fabrication d'une matrice selon la revendication précédente comprenant au moins l'étape suivante qui est effectuée après l'étape b. et avant l'étape c :
- le mélange B est maintenu sous agitation lente, à une vitesse avantageusement inférieure à 200 tours par minute, pendant une durée équivalente à une nuit et optionnellement dans lequel les étapes a, b, c, et éventuellement d, sont répétées pour former une autre zone conductrice (2) et une zone isolante (3) respectivement sur des portions dudit film.

## Patentansprüche

1. Klebende Polymermatrix (1) zur Ausgabe eines Wirkstoffes durch lontophorese, die eine erste Seite (7) aufweist, die dazu bestimmt ist, an der Haut angebracht zu werden, und eine zweite Seite (8), die dazu bestimmt ist, mit Elektroden zusammenzuwirken, **dadurch gekennzeichnet, dass** sie elektrisch leitende Zonen (2) und mindestens eine elektrisch isolierende Zone (3) umfasst, wobei jede leitende Zone (2) von einer anderen leitenden Zone (2) durch eine isolierende Zone (3) isoliert ist, und dadurch, dass die Matrix ein Gemisch umfasst, das mindestens ein Polymer und mindestens einen Klebstoff umfasst.

2. Matrix (1) nach dem vorstehenden Anspruch, wobei das mindestens eine Polymer ausgewählt wird aus: Polyurethan, Polysiloxan, Poly(methylmetacrylat), Poly(vinylalkohol), Polyethylen, Polyvinylpyrrolidon.

3. Matrix (1) nach einem der vorstehenden Ansprüche, wobei der mindestens eine Klebstoff ausgewählt wird aus: Acrylsäure, Polyacrylaten, Polyisobutylen, Polyvinylpyrrolidon, Silikonen.

4. Matrix (1) nach einem der vorstehenden Ansprüche, wobei das Gemisch mindestens einen Weichmacher umfasst, der vorzugsweise aus Sorbit, Glyzerin, Propylenglykol ausgewählt wird.

5. Matrix (1) nach einem der vorstehenden Ansprüche, wobei die Matrix eine flexible Decke ist.

6. Matrix (1) nach einem der vorstehenden Ansprüche, wobei die leitenden Zonen (2) mindestens einen abzugebenden Wirkstoff umfassen.

7. Matrix (1) nach dem vorstehenden Anspruch, wobei die leitenden Zonen (2) konfiguriert sind, um mit den Elektroden zusammenzuwirken, die einen elektrischen Strom bereitstellen, wobei der mindestens eine Wirkstoff jeder leitenden Zone (2) in Abhängigkeit von der Polarisation der Elektrode (5a, 5b) ausgewählt wird, die mit der Zone zusammenwirkt.

8. Matrix (1) nach einem der beiden vorstehenden Ansprüche, wobei die leitenden Zonen (2) unterschiedliche Wirkstoffe umfassen.

9. Matrix (1) nach einem der vorstehenden Ansprüche, wobei die mindestens eine isolierende Zone (3) eine minimale Breite von 1 mm aufweist, die die beiden leitenden Zonen (2) trennt.

10. Matrix (1) nach einem der vorstehenden Ansprüche, wobei sich die Zonen über die gesamte Dicke der Matrix erstrecken.

11. Matrix (1) nach einem der vorstehenden Ansprüche, einen Absorptionspromotor umfassend.

12. Vorrichtung zur lontophorese, umfassend mindestens zwei Elektroden (5a, 5b) und eine Energiequelle (6), die elektrisch mit den Elektroden (5a, 5b) verbunden sind, **dadurch gekennzeichnet, dass** sie eine klebende Polymermatrix (1) nach einem der vorstehenden Ansprüche umfasst.

13. Vorrichtung nach dem vorstehenden Anspruch, wobei die leitenden Zonen (2) der klebenden Polymermatrix (1) an die Elektroden (5a, 5b) angeschlossen sind.

14. Verfahren zur Herstellung einer Matrix nach einem der Ansprüche 1 bis 11, mindestens die folgenden Schritte umfassend:
a. eine Klebelösung wird in eine Polymerlösung gemischt, um ein Gemisch A zu bilden, dann
b. eine Lösung, einen Weichmacher und mindestens einen Wirkstoff umfassend, wird dem Gemisch A beigemengt, um ein Gemisch B zu bilden,
c. das Gemisch B wird danach auf mindestens einen Abschnitt eines Films, beispielsweise aus Silikon gegossen.

15. Verfahren zur Herstellung einer Matrix nach dem vorstehenden Anspruch, mindestens den folgenden Schritt umfassend, der nach dem Schritt b. und vor dem Schritt c. durchgeführt wird:
- das Gemisch B wird unter langsamem Rühren mit einer Geschwindigkeit von vorteilhafterweise weniger als 200 Umdrehungen pro Minute für eine Zeitdauer erhalten, die gleich einer Nacht ist, und wobei optional die Schritte a, b, c und eventuell d wiederholt werden, um eine weitere leitende Zone (2) und eine isolierende Zone (3) jeweils auf Abschnitten des Films zu bilden.

## Claims

1. Adhesive polymeric matrix for the delivery of an active constituent by iontophoresis with a first face (7) intended to be applied on the skin and a second face (8) intended to cooperate with electrodes, **characterised in that** it comprises electrically conducting zones (2) and at least one electrically insulating zone (3), each conducting zone (2) being insulated from another conducting zone (2) by an insulating zone (3) and **in that** the matrix comprises a mixture comprising at least one polymer and at least one adhesive.

2. Matrix (1) according to the preceding claim, wherein said at least one polymer is chosen from among: polyurethane, polysiloxane, polymethyl methacrylate, polyvinyl alcohol, polyethylene, polyvinylpyrrolidone.

3. Matrix (1) according to any one of the preceding claims, wherein said at least one adhesive is chosen from among: acrylic acid, polyacrylates, polyisobutylene, polyvinylpyrrolidone, silicones.

4. Matrix (1) according to any one of the preceding claims, wherein said mixture comprises at least one plastifier, preferably chosen from among sorbitol, glycerol, propylene glycol.

5. Matrix (1) according to any one of the preceding claims, wherein the matrix is a flexible layer.

6. Matrix (1) according to any one of the preceding claims, wherein the conducting zones (2) comprise at least one active constituent to be delivered.

7. Matrix (1) according to the preceding claim, wherein the conducting zones (2) are configured to cooperate with electrodes that output an electric current, the at least one active constituent in each conducting zone (2) being chosen as a function of the polarisation of the electrode (5a, 5b) cooperating with said zone.

8. Matrix (1) according to any one of the two preceding claims, wherein the conducting zones (2) comprise different active constituents.

9. Matrix (1) according to any one of the preceding claims, wherein the at least one insulating zone (3) has a minimum width of 1 mm separating two conducting zones (2).

10. Matrix (1) according to any one of the preceding claims, wherein the zones extend over the entire thickness of the matrix.

11. Matrix (1) according to any one of the preceding claims, comprising an absorption promoter.

12. Device for iontophoresis comprising at least two electrodes (5a, 5b) and an energy source (6) electrically connected to the electrodes (5a, 5b), **characterised in that** it comprises an adhesive polymeric matrix (1) according to any one of the preceding claims.

13. Device according to the preceding claim, wherein the conducting zones (2) of the adhesive polymeric matrix (1) are connected to the electrodes (5a, 5b).

14. Method of manufacturing a matrix according to any one of claims 1 to 11 comprising at least the following steps:
a) an adhesive solution is mixed with a polymer solution to form a mixture A, then
b) a solution comprising a plastifier and at least one active constituent is added to the mixture A to form a mixture B,
c) the mixture B is then poured on at least one portion of film, for example silicone.

15. Method of manufacturing a matrix according to the preceding claim, comprising at least the following step performed after step b and before step c:
- the mixture B is kept under slow stirring, at a speed advantageously less than 200 revolutions per minute, for a duration equivalent to one night and optionally wherein steps a, b, c and possibly d are repeated to form another conducting zone (2) and an insulating zone (3) respectively on portions of said film.
